(19) Europäisches Patentamt
European Patent Office
Office européen des brevets

(11) Publication number: **0 131 251**
**A1**

# (12) EUROPEAN PATENT APPLICATION

(21) Application number: **84107778.7**

(22) Date of filing: **04.07.84**

(51) Int. Cl.[4]: **C 12 N 11/14**
C 01 B 33/12

(30) Priority: **08.07.83 DK 3174/83**

(43) Date of publication of application:
**16.01.85 Bulletin 85/3**

(84) Designated Contracting States:
**AT BE CH DE FR GB IT LI LU NL SE**

(71) Applicant: **Superfos a/s**
**Frydenlundsvej 30**
**DK-2950 Vedbaek(DK)**

(72) Inventor: **Hansen, Jan Jorn**
**Haugesundvej 24**
**DK-28850 Naerum(DK)**

(72) Inventor: **Thygesen, Jesper Callo**
**Teglgardsvej 325 B**
**DK-3050 Humlebaek(DK)**

(72) Inventor: **Winther-Nielsen, Margrethe**
**Nymarksvaenge 18**
**DK-4000 Roskilde(DK)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath**
**Maximilianstrasse 58**
**D-8000 München 22(DE)**

(54) **Immobilized enzyme composition and process for the preparation thereof.**

(57) An enzyme composition comprising an enzyme immobilized by attachment to a particulate inorganic carrier material (2) free from calcium carbonate, calcium oxide and acid soluble components, and comprising less than 1.2% by weight of trivalent metal ions, each particle of the inorganic carrier comprising at least 90% by weight of $SiO_2$ and i) having a porosity of 25-50% by volume based on mercury porosimetry, corresponding to a void volume of about 0.15-0.45 $cm^3/g$, ii) having an average pore radius of 0.02-0.1 $\mu m$, iii) having an abrasion resistance of at least 95% when determined according to ASTM No. E 728-80 and preferably having a surface area of at least 15 $m^2/g$, based on mercury porosimetry. The carrier material may be prepared from naturally occuring inorganic materials (1) not having the desired physical and chemical properties. The enzymes may be attached to the carrier material by adsorption, optionally followed by intermolecular cross-linking, or by covalent bonding, optionally via a coupling agent.

EP 0 131 251 A1

./...

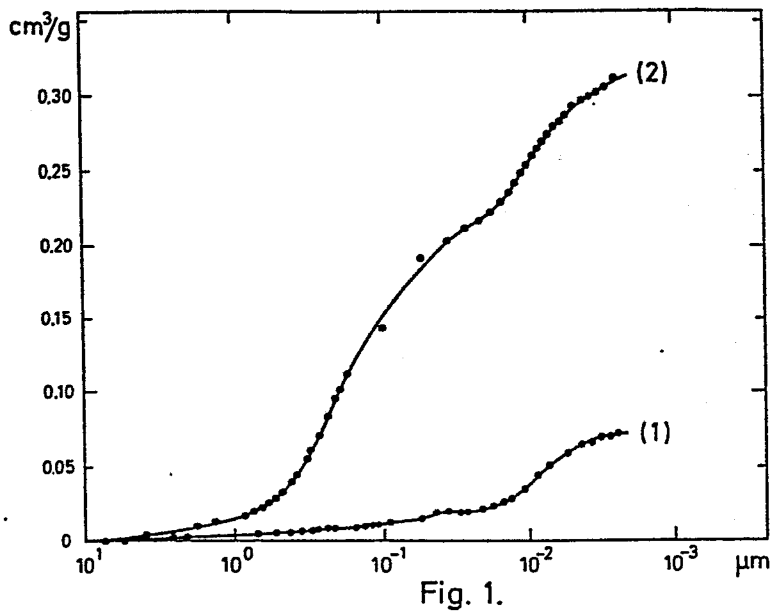


Fig. 1.

The present invention relates to an enzyme composition, a method for immobilizing an enzyme, a method for performing an enzymatic reaction, and a carrier material for enzymes.

## BRIEF DESCRIPTION OF THE INVENTION

The present invention is based on the fact that it is possible to immobilize an enzyme by adsorption, optionally followed by intermolecular cross-linking, or by covalent bonding, optionally via a coupling agent on an inorganic carrier material having advantageous physical properties. The inorganic carrier material may be prepared from a naturally occurring material without the desired properties such as chemical composition and physical properties such as porosity and surface area, by a porosity-increasing acid treatment.

## DESCRIPTION OF THE PRIOR ART

US Patent No. 3,519,538 (Messing et al.) discloses an insolubilized enzyme comprising an enzyme coupled covalently to an inorganic carrier, said carrier being i.a. a siliceous material. Specific examples of siliceous carrier materials are amorphous (glass, silica gel and colloidal silica) and crystalline (bentonite and wollastonite) materials. In the examples relating to siliceous materials, only the use of amorphous materials has been described. A porous glass is the preferred matrix. The surface of the porous glass material is cleansed with dilute $HNO_3$ solution, rinsed with distilled water, dried, and heated to about 625°C in the presence of $O_2$. Thereafter, the oxide or hydroxyl groups on the surface are reacted with an organosilane coupling agent to which the enzyme may be coupled via amide, thiourea, azo, ether, ester, and disulfide bonds. As enzymes are mentioned hydrolases, redox enzymes and transferases.

US Patent No. 3,669,841 (Miller) discloses the covalent attachment of enzymes to siliceous materials. An organosilane is held on the surface of the siliceous material, an enzyme is covalently bound to a cross-linking agent which is covalently bound to the organosilane. The enzyme may be a mixture of proteases and amylase.

US Patent No. 4,384,045 (Ho et al.) discloses a process for the preparation of a carrier for immobilization of enzymes by reacting a siliceous inorganic carrier material with a strongly basic solution and thereafter with a strongly acidic solution thereby obtaining a very high number of reactive hydroxyl groups on the surface. Silica gel is mentioned as a suitable carrier material. It is possible to immobilize enzymes on the surface of the carrier material by covalent bonding to hydroxyl groups pendant from the surface of the carrier, and it is possible to obtain multiple "layers" of immobilized enzyme. The enzymes are bound to a silylated surface via a difunctional coupling agent, and lactase is the only enzyme mentioned in the claims.

## DETAILED DESCRIPTION OF THE INVENTION

The enzyme composition according to the invention comprises the enzyme immobilized by attachment to a particulate inorganic carrier material substantially free from calcium carbonate, calcium oxide and other acid soluble components, and comprising less than 1.2% by weight of trivalent metal ions, each particle of the inorganic carrier comprising at least 90% by weight of $SiO_2$ and

i) having a porosity of 25-50% by volume based on mercury porosimetry, corresponding to a void volume of about 0.15-0.45 $cm^3/g$ as determined by mercury porosimetry

ii) having an average pore radius of 0.02-0.1 μm

iii) having an abrasion resistance of at least 95%, preferably about 99%, as determined according to ASTM No. E 728-80.

A preferred carrier material comprises at least 95% by weight of $SiO_2$.

A preferred carrier material to be used in the enzyme compositions is a material with a surface area of at least 15 $m^2/g$, preferably at least

25 $m^2/g$, and more preferably at least 35 $m^2/g$, based on mercury porosimetry.

In the carrier material, pores with radia of 0.005-1 µm account for at least 80%, preferably at least 90% of the porosity.

The carrier material may be used in any particle size suitable for the process in which the immobilized enzyme is involved. Depending on the use, suitable particle sizes are: less than 50 µm, 0.1-0.5 mm, 0.5-1 mm, 1-2 mm, 1.5-2 mm, 1.5-2.5 mm, 2-4 mm and 1-6 mm.

The carrier material comprising at least 90% of $SiO_2$ has a hardness of about 7 on the Mohs' scale.

The carrier material can be prepared from a naturally occurring inorganic material comprising 20-50% of $CaCO_3$ and 80-50% of $SiO_2$, being partly crystalline and having an X-ray diffraction pattern with the following positions of the principal reflection peaks (in planar spacings, d(Å)): 4.328, 4.267, 4.092, 4.053, 3.860, 3.349, 3.040, 2.497, 2.283, 2.093, 1.912, 1.873, and furthermore

i) having a porosity of at least 10%, preferably at least 20%, by volume based on mercury porosimetry, corresponding to a void volume of about at least 0.05 $cm^3/g$, preferably at least 0.10 $cm^3/g$, as determined by mercury porosimetry

ii) having a broad pore radius distribution with pore radia from less than 0.0025 µm to about 5 µm, as determined by mercury porosimetry

iii) having a surface area of at least 10 $m^2/g$, preferably at least 16 $m^2/g$, based on mercury porosimetry,

by porosity-increasing treatment.

In a special aspect of the invention, the carrier material is identical to a material prepared from an inorganic raw material having the

following X-ray diffraction pattern expressed by relative intensities as a function of the interplanar spacings ($I/I_{max}$, d(Å)): 23, 4.328; 25, 4.267; 35, 4.092; 36, 4.053; 15, 3.860; 4, 3.792; 54, 3.349; 100, 3.040; 4, 2.846; 1, 2.533; 23, 2.497; 5, 2.478; 5, 2.460; 23, 2.283; 2, 2.236; 4, 2.130; 20, 2.093; 2, 1.979; 7, 1.924; 20, 1.912; 22, 1.873; 7, 1.818, by porosity-increasing treatment.

The raw material from which the carrier material can be prepared is a naturally occurring material consisting of silicified limestone nodules. Said material has been formed by a silification process in calcareous sediments. The silica has been precipitated as a metastable mineral in the silification process resulting in a material which may be characterized as calcareous opal-CT flint. It is contemplated that the silica component comprises quartz, opal, cristoballite, and tridymite.

The chemical composition of the naturally occuring starting material (based on atomic absorption analysis and calculated as oxides; % by weight) is

| | |
|---|---|
| $SiO_2$ | 50-80 |
| CaO | 11-28 |
| $Na_2O$ | 0.1-0.2 |
| $K_2O$ | 0.1-0.2 |
| MgO | 0.1-0.2 |
| $Fe_2O_3$ | 0.1-0.8 |
| $Al_2O_3$ | 0.4-1.2 |

The CaO content corresponds to a calcium carbonate content of about 20-50%, preferably about 30-35% by weight of $CaCO_3$. It is preferred to use a naturally occurring starting material containing (calculated as oxides; % by weight) about 60-65 $SiO_2$, preferably about 63 $SiO_2$, and 15-20 CaO, preferably about 19 CaO, the latter corresponding to a content of $CaCO_3$ of about 34% by weight.

The naturally occuring starting material has the following X-ray diffraction pattern expressed by relative intensities as a function of the interplanar spacings ($I/I_{max}$, d(Å)): 23, 4.328; 25, 4.267; 35,

4.092; 36, 4.053; 15, 3.860; 4, 3.792; 54, 3.349; 100, 3.040; 4, 2.846; 1, 2.533; 23, 2.497; 5, 2.478; 5, 2.460; 23, 2.283; 2, 2.236; 4, 2.130; 20, 2.093; 2, 1.979; 7, 1.924; 20, 1.912; 22, 1.873; 7, 1.818, and Fig. 2 shows a typical X-ray diffractogram.

The porosity of the naturally occuring starting material is about 20% by volume when measured by water absorption (physical test methods are described below) corresponding to a void volume of about 0.10 $cm^3/g$.

The naturally occuring starting material per se is not suitable for use as a carrier material for enzyme immobilization due to the $CaCO_3$ content and insufficient physical properties (porosity and surface area).

The naturally occuring starting material has pore radia from less than 0.0025 µm to about 5 µm with a pore radius distribution maximum of about 0.01 µm and an average pore radius of about 0.02 µm.

The surface area can be calculated to be at least 10 $m^2/g$ based on mercury porosimetry, preferably at least 16 $m^2/g$.

Using the BET method the surface area can be calculated to be at least 5 $m^2/g$, preferably at least 9 $m^2/g$.

The apparent density of the starting material is about 2.5 $g/cm^3$.

Based on measurements of isothermal water adsorption at different relative vapour pressures $p/p_o$ less than 1 it can be concluded that pores with radia of less than 0.04 µm account for about 64% by volume of the total pore volume.

The starting material used for the preparation of the porous carrier material differs essentially from other materials to be used as enzyme carriers, and the process for the preparation of the carrier material is a special process.

The carrier material may be prepared from a naturally occurring material found as relatively big stones having a diameter of up to about 40 cm. This material is subjected to a porosity-increasing treatment, i.e. an acid treatment after an optional size-reduction treatment of the particles with subsequent screening and selection of a suitable fraction, e.g. of 0.01-10 mm. The size reduction may be carried out by jaw crushing, ball milling, etc. After the size reduction (disintegration), the material is subjected to a screening process, e.g. on a system of sieves, followed by selection of the desired fraction(s).

It is preferred to carry out the porosity-increasing acid treatment on a material with a particle size of at the most 10 mm, especially at the most 6 mm, preferably at the most 2 mm.

Thereafter, the crushed particles are subjected to an acid under conditions sufficient to dissolve substantially all $CaCO_3$ contained in the starting material. Suitable acids are strong mineral acids and organic acids, preferably nitric acid, hydrochloric acid and acetic acid.

As $CaCO_3$ in the crushed material is readily dissolved, it is most convenient to use only a slight excess of acid. Together with the content of $CaCO_3$, other acid-extractable components are removed, leading to a carrier material with a reduced content of e.g. trivalent metal ions. The carrier material prepared by acid treatment consists of at least 90%, preferably at least 95%, and more preferably about 99% by weight of $SiO_2$.

The amount of acid employed is preferably 1-1.5 times the stoichiometric amount, especially about 1.2 times the stoichiometric amount, calculated on the basis of the $CaCO_3$ content.

The acid treatment is to be performed at a temperature which provides a reasonable duration of the treatment, elevated temperatures being preferred. Temperatures of about 20-90°C are preferred, especially about 80°C.

Complete reaction is indicated by the absence of $CaCO_3$, and analysis for $CaCO_3$ may be performed by e.g. X-ray diffraction analysis or atomic absorption analysis.

Subsequent to the acid treatment, the material obtained is separated from the liquid phase containing the salts of the acid involved, and thereafter rinsed to yield a carrier material which is substantially free from acid residues and cations. After the rinsing process, which is normally carried out with water, the material is dried.

The acid treatment results in a loss of weight of about 20-50%, normally about 25-40%, especially about 30-35%.

Owing to the dissolution of $CaCO_3$ during the acid treatment, the pore volume increases to form a material having a porosity of 25-50% by volume, preferably 30-45% by volume, more preferably 40-45% by volume and especially about 42% by volume based on mercury porosimetry, corresponding to a void volume of about 0.15-0.45 $cm^3/g$, preferably 0.18-0.36 $cm^3/g$, more preferably 0.29-0.36 $cm^3/g$ and especially about 0.32 $cm^3/g$ (when measured by mercury porosimetry) in combination with excellent mechanical strength. Owing to the mechanical strength the carrier material may be used in tall columns.

The acid treatment yields a material having pore radia from 0.001 µm to 10 µm with pore radius distribution maxima at about 0.01 µm and at about 0.2 µm. The average pore radius is 0.02-0.1 µm, especially 0.03-0.05 µm, preferably 0.05 µm. Pores with radia of 0.005-1.0 µm account for at least 80%, preferably at least 90% of the porosity.

The acid treatment yields a material with a surface area of at least 20 $m^2/g$, preferably at least 30 $m^2/g$, more preferably about 40-45 $m^2/g$, based on mercury porosimetry.

The surface area according to the BET method is at least 10 $m^2/g$, preferably at least 19 $m^2/g$.

The carrier material has an apparent density of 2.3 $g/cm^3$ and a bulk density of 0.6-0.7 $g/cm^3$.

The carrier material to be used in the enzyme composition according to the invention differs from e.g. the material described in US Patent No. 3,519,538 in being a mixture of crystalline $SiO_2$ and amorphous opal.

The porosity-increasing treatment yields, unpredictably, a carrier material with a pore size which is advantageous for the immobilization of enzymes. The average pore radius of 0.02-0.1 µm, especially 0.03-0.05 µm, preferably 0.05 µm, and the fact that pores with radia of 0.005-1 µm in the carrier material account for at least 80% and preferably at least about 90% of the porosity, offers advantageous possibilities for diffusion of products and substrates.

After the porosity-increasing treatment the carrier material was shown to contain silanol groups on the surface offering the possibility of binding an advantageous amount of enzymes on the surface of the pores.

Immobilizing enzymes is a well-known technique offering several advantages as 1) it is possible to achieve repeated use of the enzymes involved and enzyme stability is often enhanced, 2) separation of product and enzyme is facilitated and there is reduced contamination with enzyme in the end product and 3) it implies better possibilities for reactor design (as e.g. fluid bed and column reactors are feasible).

The enzyme may e.g. be attached to the carrier by adsorption, optionally followed by intermolecular cross-linking, or by covalent bonding, optionally via a coupling agent. The term "adsorption" indicates the binding of a substance to the surface of another substance. By this is meant that the enzyme is non-covalently bound directly to the surface of the carrier material, partly by means of hydrogen bonds and partly by ionic bonds between the enzyme and the silanol groups of the carrier material.

Methods and reagents for the binding of enzymes to carrier materials are in principle known, but the combination with the carrier material

according to the invention is novel. Furthermore, adsorption of functionally active enzymes directly to the surface of siliceous surfaces, optionally followed by a cross-linking, has not been described in the patent specifications mentioned in the description of the prior art.

The carrier material (the immobilizer) comprises silanol groups on the surface, said groups acting as functional groups, to which a coupling agent for the enzyme or the enzyme itself may be attached. The number of silanol groups is estimated to be about 2.2 per 100 Å$^2$ of the carrier material described here. The number of silanol groups on the surface may be determined by neutralization with calcium hydroxide according to the reaction scheme:

$$\equiv Si\text{-}OH + Ca(OH)_2 \rightarrow \equiv SiO^- Ca^{++} OH^- + H_2O$$

Enzymes which may be attached to the inorganic carrier material may be any enzyme selected from the group consisting of oxidoreductases, transferases, hydrolases, lyases, isomerases and ligases, according to the classification system of the International Union of Biochemistry.

The attachment to the carrier material may be performed by an adsorption and preferably followed by a cross-linking of the enzyme molecules. The cross-linking agent may be selected depending on the enzyme employed, and an example of cross-linking agents is glutardialdehyde.

The attachment of the enzyme to the carrier material may also be obtained via covalent bonds between the enzyme and the inorganic carrier material, and in one embodiment thereof, the covalent bonding is performed via a coupling agent.

As a coupling agent may be mentioned cyanogen bromide reacting as follows

$$\text{carrier}\left\{\begin{matrix}\text{-OH}\\\text{-OH}\end{matrix}\right. + CNBr \rightarrow \text{carrier}\left\{\begin{matrix}\text{-O}\\\text{-O}\end{matrix}\right\rangle C{=}NH \ (+ HBr)$$

The cyanogen bromide activated carrier may react with active hydroxyl or amino groups in the enzyme.

Another way of attaching enzymes to the carrier material is to silylate the carrier material by means of a silylating agent such as (3-aminopropyl)triethoxysilane. Subsequent to the silylation, the carrier material may be treated with a coupling agent such as glutardialdehyde. The reaction may be illustrated as follows (other reaction schemes are feasible, depending on the exact reaction conditions):

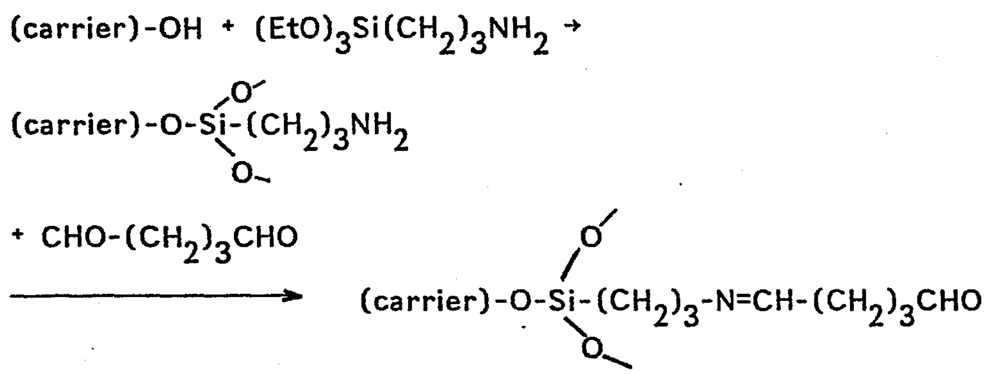


The silylated carrier material treated with coupling agent may then be reacted with an amino group in the enzyme.The reaction may be depicted as follows (other reaction schemes are feasible, depending on the exact reaction conditions):

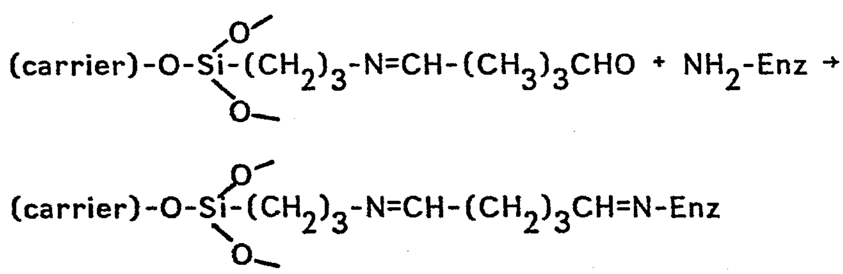

Depending on the coupling agent employed, different reactive groups on the enzymes may be attached to the carrier material derivatized with reactive terminal groups.

As examples of reactive groups on the enzymes may be mentioned e.g. amino groups, carboxy groups, aliphatic and aromatic hydroxy groups, and mercapto groups, and as examples of reactive terminal groups on the carrier material may be mentioned, but not limited to, amino groups, carboxy groups, acyl halide groups, alkyl halide groups, mercapto groups, etc. Illustrative examples of reactive groups on the enzymes (X), reactive terminal groups on the carrier materials (Y) and coupling agents are as follows:

| Enz-X<br>X | Carrier-Y<br>Y | Coupling Agent |
|---|---|---|
| $NH_2$ | $NH_2$ | Glutardialdehyde, triazines, iso(thio)cyanates |
| COOH | $NH_2$ | Carbodiimides |
| $NH_2$ | COOH | Carbodiimides or via acid chloride or via azide |

Preferred enzymes to be immobilized on the carrier material according to the invention are acylases, especially amidases and esterases, and decarboxylating enzymes.

As preferred examples may be mentioned amidases, an example thereof being amino acylase (E.C.3.5.1.14) catalyzing the reaction

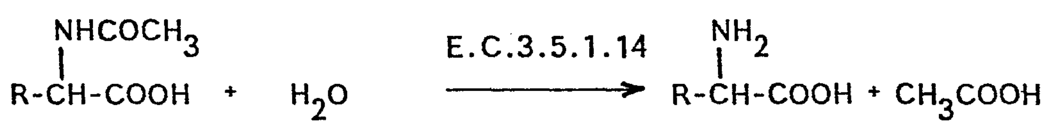


Another important example of acylases is penicillin acylase which is used in the synthesis of penicillins.

Further important enzymes are lyases and glucose isomerases.

An especially interesting enzyme is acetolactate decarboxylase (E.C.4.1.1.5) which catalyzes the following reaction:

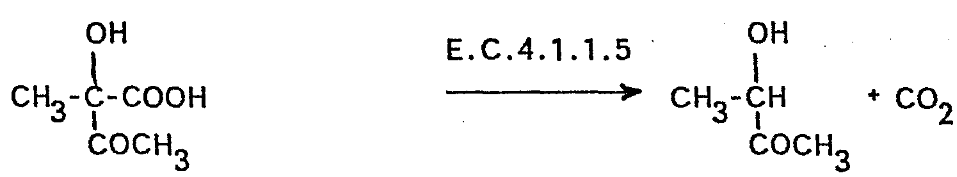


thereby removing acetoloctate which is a precurser for diacetyl which in large concentrations conveys an objectionable character to certain biochemical products (Godtfredsen, S.E., and Ottesen, M., *Carlsberg Res. Commun.* 47, 1982, p. 93.)

DESCRIPTION OF FIGURES

Fig. 1 shows the results of mercury porosimetry on the naturally occurring starting material (curve (1)) and on the inorganic carrier material (curve (2)). The pore volume ($cm^3/g$) in plotted against the pore radius (µm, indicated in logarithmic scale). In the calculation of pore radia, it was assumed that there was a contact angle of 140° between mercury and the carrier material. It appears that the pores in the carrier material are divided into two groups having pore radius distribution maxima of about 0.01 and of about 0.2µm, respectively.

Fig. 2 shows the X-ray diffractogram of the starting material for the carrier material.

Fig. 3 shows the X-ray diffractogram of the carrier material.

Fig. 4 is a SEM photo of the naturally occuring starting material.

Fig. 5 is a SEM photo of the carrier material.

PHYSICAL METHODS

*Mercury Porosimetry*

Mercury porosimetry is a suitable test method for the determination of the pore size distribution. When the contact angle of a liquid to a solid is above 90° (mercury: about 140°), only a minimum of pressure (p) is necessary in order to force the liquid into a cylindrical cavity having the radius r. (According to a reference to Bond, R.L.: *Porous Carbon Solids*, Academic Press, London and New York, 1967):

$$p = \frac{-2\delta\cos\theta}{r}$$

wherein $\delta$ = the surface tension of the liquid
$\theta$ = the contact angle (if > 90°)

The pore size distribution may in principle be measured by forcing such a liquid into pores of decreasing size by increasing the pressure and determining the amount, V, of the penetrating liquid as a function of p. Provided that the pores are cylindrical, r may be calculated as a function of the pressure p. When V is plotted against r, the pore volume distribution $\frac{dV}{dr}$ may be determined as a function of r by graphical differentation.

The total internal surface area S of all pores with radii between $r_a$ and $r_b$ ($r_a > r_b$) may be calculated by the equation

$$S = \sum_{i=a}^{b} \left( \frac{2 \times (v_{i+1} - v_i)}{0.5\,(r_{i+1} + r_i)} \right)$$

wherein for cylindrical pores

$$S_p = \frac{2\,V_p}{r_p}$$

wherein $S_p$ = the surface of the pore
$V_p$ = the volume of the pore
$r_p$ = the radius of the pore

The determinations of mercury porosimetry on the carrier material were performed on a Mercury Penetration Porosimeter, Model 905-1, Micromeritics Instrument Corporation, Norgrass, Georgia, USA.

A sample of 3-4 g was used for each test run. In calculating the pore radius r corresponding to the pressure p, $\delta$ = 484 dyn/cm and $\theta$ = 140° were used. (140° is the normal contact angle between mercury and solids). Fig. 1 shows the curve illustrating pore volume plotted against pore radius, measured by mercury porosimetry.

*Determination of the surface area according to the BET method*

The BET method (according to a reference to Nelson, F.M. and Eggertsen, F.T. *30*, 1958, p. 1387) was used in the calculation of the specific surface area. Nitrogen at -196°C was used as adsorbant.

*Isothermal adsorption of water*

Isothermal water adsorption on the starting material was carried out at different relative vapour pressures $p/p_o$ less than 1 leading to the following connection between the amount of water adsorbed per 100 g of solid and the relative vapour pressure (g/100 g, $p/p_o$): 0.0, 0.11; 0.0, 0.23; 0.5, 0.58; 1.2, 0.75; 1.6, 0.83; 4.0, 0.92; 6.9, 0.97.

*Density and water absorption*

The apparent density and water absorption were determined in principle according to Danish Standard No. 405.2, using an evacuation step in order to accelerate the absorption of water in the pores.

The porosity determined by water absorption shows a larger value than the determination by mercury porosimetry caused by the fact that water may penetrate into pores too small for mercury penetration at the pressure used (50,000 psia).

*Determination of average pore radius*

The average pore radius was calculated by the equation

$$r_{av} = \frac{2 \times v}{s_{BET}}$$

wherein $v$ = the pore volume as determined by water absorption
and $s_{BET}$ = surface area according to the BET method

*X-ray diffractograms*

X-ray diffractograms were made using $CuK\alpha_1$-radiation. The diffraction analysis was performed at a rate of 1/4° per minute or 1° per minute and with a time constant of 8 sec or 2 sec, respectively. The amount of impulse was plotted against the diffraction angle 2 θ. The relative intensities were calculated as the ratio between the individual heights of maxima and the height of the maximum with the maximum intensity in the spectrum. The values of the interplanar spacings were calculated according to the formula:

$$d = \frac{\lambda_{CuK\alpha_1}}{2\sin\theta}$$

$\lambda_{CuK\alpha_1}$ being 1.54051 Å and
θ the angle between the direction of the entering rays and the planes.

The analyzed material was identified by comparison between the calculated planar interspacings d and relative intensities with standard tables (ASTM) and data in the literature.

Preparation of test sample for X-ray diffractometry

About 1 g of the material was disintegrated in an agate ball mill until a fine powder was obtained (15 minutes).

*Determination of silanol groups*

Silanol groups on the surface of the carrier material may be determined according to the scheme:

$$\equiv Si\text{-}OH + Ca(OH)_2 \rightarrow \equiv Si\text{-}O^- Ca^{++} OH^- + H_2O$$

From a saturated and filtrated solution of $Ca(OH)_2$ at about 30°C, 12 different $Ca(OH)_2$-solutions were prepared, the concentrations varying from 0.6 to 1.0 g of CaO per liter. The exact concentrations were

determined by atomic absorption. Samples of about 1.5 g of carrier material were placed in polyethylene flasks and covered with nitrogen. 50 ml of $Ca(OH)_2$-solution were added. The flasks were shaken vigorously for 3 minutes, and the conductivity (L) was measured. Based upon the conductivity in the individual $Ca(OH)_2$-solutions and the known concentrations $C_o$, the equilibrium concentration $C_1$ could be calculated as

$$C_1 = \frac{L_1}{L_o} \times C_o$$

When the amount of CaO (gram) absorbed per gram of carrier material was plotted against $C_1$ (mol/liter), the maximum amount of CaO absorbed was found to be 0.004 g per gram of carrier material. This corresponds to 7.1 meqv. of OH per 100g of carrier material. By means of the surface area ($19 m^2$ per g of carrier material) according to the BET method and Avogadros number, the number of silanol-groups per 100 $Å^2$ of carrier material may be calculated as 2.2 silanol-groups per 100 $Å^2$.

ASSAYS

Aminoacylase (obtained from Sigma as a freeze-dried product prepared from hog kidney), was assayed at 25°C in 3.0 ml of 15 mM N-acetyl-L-methionine in 20 mM phosphate puffer (pH 7.0). The immobilized enzyme was shaken on a Griffin shaker during the assay. After 15 minutes, a sample of the liquid phase was taken out, and the free amino acid formed was determined by the ninhydrin method (Moore, S. and Stein, W.H., *J.Biol.Chem. 176*, 1948, p. 367). After terminated assay, the immobilized enzyme was filtered off, dried at 115°C overnight and weighed. One IU was defined as the amount of aminoacylase which in this assay forms 1 µMol L-methionine per minute.

Acetolactate decarboxylase in dissolved form was determined as described (Godtfredsen, S.E. and Ottesen, M., *Carlsberg Res. Commun. 47*, 1982, p. 93). Immobilized acetolactate decarboxylase was similarly

assayed on a shaking table and immobilized *aminoacylase* was used as a blind. After the assay was terminated, immobilized enzyme was filtered off and washed, dried overnight at 115°C and weighed. One U acetolactate decarboxylase formed 1 µMol acetoin per hour at 37°C and pH 6.5 from acetolactate.

Protein was determined according to Lowry et al., (*J. Biol. Chem. 193*, 1951, p. 265) with Bovine Serum Albumin as standard.

EXAMPLE 1

*Preparation of the carrier material*

Ground-down, raw calcareous opal CT-flint stones were treated with an excess of acid, whereby the content in the stones of $CaCO_3$ (34% of $CaCO_3$ on an average) was dissolved.

Three fractions of ground-down calcareous opal CT-flint stones (grain sizes < 1/2, 1/2-2 and 3-5 mm) have been treated with various acids with and without heating and for various periods of time.

Samples of the ground-down stones were dried in an incubator at 100°C for 48 hours. About 2 g of material were weighed out in beakers and an excess of (minimum 25 ml/g of stone) 0.4M acid ($HNO_3$, HCl or $CH_3COOH$) was added. The beakers with stone and acid were left for treatment at room temperature for periods varying from 1/2 to 4 hours. Stirring with glass spatula was done frequently. The samples treated with hot acid were heated on electrical plates to a temperature of about 80°C with frequent stirring.

The individual samples were filtered on a glass filter, washed with distilled water until pH 6 and left to dry on the filter in an incubator at 110°C until constant weight. The loss in weight was calculated.

X-ray diffraction analysis was carried out on the acid-treated samples wherein the loss in weight was ≥ 34% to ascertain any content of $CaCO_3$.

The porosity was determined in the carrier material by means of mercury porosimetry. The results are shown in Fig. 1, curve (2). The surface area was 19 $m^2/g$ according to the BET method.

## TABLES

Loss of weight (%, based on the original amount of sample) after treatment with acids carried out on 3 size fractions, i.e.
a: particles of less than 0.5 mm, b: particles of 0.5-2 mm, and
c: particles of 3-5 mm.
M = mean value of duplicate, S = standard deviation (%)

Table a

| Treatment | Loss of weight: 2M or 0.4M $CH_3COOH$ | | 0.4M HCl | | 0.4M $HNO_3$ | | X-ray diffraction ** |
|---|---|---|---|---|---|---|---|
| | M | S(%) | M | S(%) | M | S(%) | (1°/min) |
| 0.5 h at room temperature | 23 | 7 | 34 | 6 | | | |
| 1 h at room temperature | 33 | 3 | 34 | 0 | 35 | 0 | no reflections for $CaCO_3$ |
| 2 h at room temperature | 34 | 0 | 34 | 1 | 35 | 8 | no reflections for $CaCO_3$ |
| 4 h at room temperature | 34 | 0 | 35 | 1 | | | |
| 1 h at 80°C | 34* | | 34* | | 36 | 2 | |

* single determination
** results after $HNO_3$-treatment

Table b

| Treatment | Loss of weight 0.4M $HNO_3$ M | S(%) | X-ray diffraction Relative intensity of $CaCO_3$-reflection* |
|---|---|---|---|
| 2 h at room temperature | 35 | 1 | 11 |
| 2 h at 80°C | 37 | 0 | 3 |
| 4 h at 80°C | 38 | 0 | 0 |
| Untreated starting material | | | 100 |

* $2\theta = 29.4°$

Table c

| Treatment | Loss of weight 0.4M HCl or 0.4 M $HNO_3$ M | S(%) | X-ray diffraction Relative intensity of $CaCO_3$-reflection* |
|---|---|---|---|
| 1 h at room temperature | 18 | 3 | |
| 2 h at room temperature | 21 | 16 | |
| 4 h at room temperature | 25 | 1 | |
| 1 h at 80°C | 24 | 11 | |
| 4 h at 80°C | 34 | 4 | 20 |
| Untreated starting material | | | 100 |

* $2\theta = 29.4°$

EXAMPLE 2

*Pre-treatment of carrier material*

In order to secure against batch-to-batch variations, the carrier material (300 g, 0.5-1 mm) (the preparation of which is described in Example 1) was heated with 5% $HNO_3$ (1250 ml) to 80°C for 1 hour during frequent shaking The carrier material was filtered off, thoroughly washed with water and dried at 115°C overnight. This acid-washed carrier material was used in all subsequent silylations and enzyme immobilizations.

EXAMPLE 3

*Silylation*

(3-Aminopropyl)-triethoxysilane (Dynamit Nobel) was distilled (b.p. 109-110°C/12 mm Hg) and used as described by Weetall, H.H., *Methods Enzymol. 44*, 1976, p. 134, for silylation in an aqueous medium and in toluene with varying amounts of silane. The reaction conditions appear from Table I (vide p. 25).

EXAMPLE 4

*Immobilization of acetolactate decarboxylase (E.C.4.1.1.5) on silylated carrier material via coupling with glutardialdehyde:*

To silylated (I or V) carrier material (4.0 g) was added 2.5% glutardialdehyde (80 ml) in 50 mM phosphate buffer (pH 6.5). The mixture was kept at room temperature with frequent shaking whereby the carrier material gradually assumed a tan to reddish brown colour. A few per cent of the particles clearly differed from the rest in that they were almost white and apparently did not react with glutardialdehyde. After reaction for 3 hours, the mixture was filtered and the product washed thoroughly with water.

The moist material was added to an enzyme solution (10 ml) containing acetolactate decarboxylase (2440 U), protein (106 mg) in 60 mM phosphate buffer, pH 6.5, and the mixture was left with frequent shaking at room temperature for 5 hours. The reddish brown immobilisate was filtered off and washed on the filter with 0.2 M phosphate buffer (80 ml, pH 6.5) in order to remove adsorbed enzyme, and then washed with water (110 ml). The immobilized enzyme was stored moist in a closed container.

EXAMPLE 5

*Immobilization of acetolactate decarboxylase (E.C.4.1.1.5) on on silylated carrier material via coupling with 1-cyclohexyl-3-(2-morpholinoethyl)carbodiimide, metho-p-toluenesulphonate (CMC)*

To silylated (V) carrier material (3.0 g) was added an enzyme solution (13 ml) (containing acetolactate decarboxylase (880 U), protein (40 mg) in 0.15 M phosphate buffer, pH 4.0) followed by CMC (500 mg). The reaction mixture was kept at 4°C overnight, but quickly started to give an amorphous precipitate. The next day, the supernatant (pH 6.0) and the amorphous precipitate were decanted off. The carrier material was filtered and washed on the filter with 0.2 M phosphate (50 ml, pH 6.5) followed by water (300 ml), whereupon the product was stored moist in a closed container.

EXAMPLE 6

*Adsorption of acetolactate decarboxylase (E.C.4.1.1.5) on non-silylated carrier material followed by cross-linking with glutardialdehyde*

To an enzyme solution (8.6 ml) (containing acetolactate decarboxylase (2280 U) and protein (58 mg)) dialyzed against water was added non-silylated carrier material (3.0 g) and the solution was kept at 4°C overnight whereupon the adsorbed enzyme was filtered off and washed with water (75 ml).

The moist material was divided into two portions of approximately equal size which were separately added to 20 ml of a 0.5% and a 2.5% aqueous solution of glutardialdehyde, respectively. After 2 hours at room temperature with frequent shakings, the two reaction mixtures were separately filtered and washed with 0.2 M phosphate buffer (100 ml, pH 6.5) and water (200 ml). The two immobilized enzyme compositions were stored moist in closed containers.

EXAMPLE 7

*Immobilization of aminoacylase (E.C.3.5.1.14) on silylated carrier material via coupling with glutardialdehyde*

To 50 mM phosphate buffer (90 ml, pH 7.0) was added aqueous 25% glutardialdehyde (10 ml) and silylated (VI) carrier material (5.0 g). After 2 hours at room temperature with frequent shaking, the now reddish brown material was filtered off and thoroughly washed with water. The moist material was added to an enzyme solution containing aminoacylase (6000 IU), and protein (250 mg) in 20 ml 50 mM phosphate buffer (pH 7.0) and stirred at room temperature for 5 hours. The immobilized enzyme was filtered off and washed first with 0.1 M phosphate buffer (60 ml, pH 7.0) and then with water (300 ml). The filtrate and the washings contained protein (185 mg) and aminoacylase (3360 IU). The immobilized enzyme was stored moist in a closed container, and was found to contain 13 mg of protein per gram and an enzymatic activity of 2.9 IU per gram.

RESULTS AND DISCUSSION

Silylation

The silylation experiments carried out are listed in Table I. The silylation reagent will react with silanol groups present on the carrier material, and it appears that under the conditions applied the carrier

material can covalently attach up to 7% of its weight in silane. The amounts of bound silane as determined by difference weighing were in all cases about half the amounts of bound silane as determined as non-recovered silane. This may be due to loss of residual water from the carrier material during the silylation. Silylation (VII) and (VIII) were control experiments showing that no procedural loss of silane occurred, and that silica 60 (Merck) compared to the carrier material could bind ≥ about 10 times as much silane per gram.

Silylation (I) carried out in an aqueous medium seemed to bind a considerably smaller amount of silane than could be obtained by silylation in toluene. This is in agreement with previous observations on other inorganic carriers, (Weetall H.H, *Methods Enzymol. 44*, 1976, p. 134).

Immobilization of Acetolactate Decarboxylase and Aminoacylase

Acetolactate decarboxylase can be found in a number of bacteria (Chuang, L.F. and Collins, E.B., *J. Bacteriol. 95*, 1968, p. 2083; Juni, E., *J. Biol. Chem. 195*, 1952, p. 715; Godtfredsen, S.E. et al., *Carlsberg Res. Commun. 48*, 1983, p. 239) and purified as described in the literature (Løken, J.P. and Størmer, F.C., *Eur. J. Biochem. 14*, 1970, p. 133). A partial purification (Godtfredsen, S.E. and Ottesen, M., *Carlsberg Res. Commun. 47*, 1982, p. 93) furnished the enzyme as an ammonium sulphate precipitate, which was dialyzed either against distilled water or against 25 mM phosphate (pH 6.5). The two dialyzed enzyme solutions containing 6.8 mg/ml protein with 39 U per mg of protein and 13.2 mg/ml protein with 23 U per mg of protein, respectively, were both used for the immobilization experiments as described in Examples 4, 5, and 6.

The results of the immobilization experiments with acetolactate decarboxylase are listed in Table II. The coupling with glutardialdehyde was performed with both silylated (V) carrier material which showed the highest degree of silylation (Table I) and with silylated (I) carrier material, which had been silylated in an aqueous medium and only seemed to be silylated to a small extent. Both of the silylated carrier

material compositions assumed a tan to reddish-brown colour during the glutardialdehyde reaction, although silylated (V) carrier material clearly was the more strongly coloured. Both of the two silylated carrier material compositions gave enzymatically active products during the glutardialdehyde coupling, but activity as well as yield and protein binding were higher for the carrier material silylated in aqueous medium.

The CMC coupling did not seem to be suitable for acetolactate decarboxylase. Although the activity losses during pH adjustment and washing were smaller than was observed for aminoacylase, the immobilisate was practically devoid of enzymatic activity.

The adsorption on non-silylated carrier material followed by glutardialdehyde cross-linking gave compositions of immobilized acetolactate decarboxylase with good activity. 79% of the added protein amount and 89% of the added activity were adsorbed, and after cross-linking, both activity and yield were the highest obtained. It did not seem to be of great importance whether the cross-linking took place in 0.5% or in 2.5% glutardialdehyde.

The immobilization of aminoacylase to silylated carrier material via coupling with glutardialdehyde gave an enzymatically active immobilisate, albeit in low yield (0.5%) and with recovery of 56% of the starting activity in the washings.

After the immobilizations, the carrier material was washed with a high buffer concentration to liberate adsorbed protein. The immobilized enzyme must therefore be considered to be covalent attached, and this is supported by the fact that for the immobilized aminoacylase, no leakage of enzyme to the solution under assay conditions was observed, and removal of immobilized enzyme by filtration effectively removed all enzymatic activity.

For aminoacylase as well as for acetolactate decarboxylase, both coupling with glutardialdehyde and adsorption plus glutardialdehyde cross-linking gave an attachment of 11-18 mg of protein per gram of carrier material.

Table I

Silylation of Carrier Material

| Silylation | Material | Amount Silane | Solvent | Temperature |
|---|---|---|---|---|
| I | 20 g carrier | 5.5 ml | 195 ml water, adj. to pH 3.8 | 80°C |
| II | 20 g carrier | 2.0 ml | 75 ml toluene | reflux |
| III | 20 g carrier | 5.0 ml | 100 ml toluene | reflux |
| IV | 20 g carrier | 10.0 ml | 100 ml toluene | reflux |
| V | 20 g carrier | 10.0 ml | 100 ml toluene | reflux |
| VI | 41 g carrier | 10.0 ml | 100 ml toluene | reflux |
| VII | 0 | 5.0 ml | 100 ml toluene | reflux |
| VIII | 10 g silica 60 | 4.9 ml | 100 ml toluene | reflux |

| Silylation | Reaction time | Recovered silane a) | Silane bound per gram of carrier b) | Weight increase per gram of carrier during silylation |
|---|---|---|---|---|
| I | 3 hr | - | - | 3.5 mg |
| II | 22 hr | 58% | 40 mg | 19 mg |
| III | 15 hr | 23% | 40 mg | 21 mg |
| IV | 17 hr | 89% | 50 mg | >15 mg |
| V | 42 hr | 86% | 67 mg | 31 mg |
| VI | 70 hr | 79% | 49 mg | 19 mg |
| VII | 18 hr | 99% | - | - |
| VIII | 18 hr | 1.5% | 433 mg | 179 mg |

a) The carrier silylated in toluene was filtered off and washed with toluene and acetone. The filtrate and washings were evaporated in vacuo, dissolved in water and titrated with hydrochloric acid. The silylated carrier was dried at 115°C overnight.

b) Calculated from non-recovered silane.

Table II

Immobilization of acetolactate decarboxylase
A = Glutardialdehyde coupling on silylated (I) carrier material (vide Table I)
B = Glutardialdehyde coupling on silylated (V) carrier material (vide Table I)
C = CMC coupling on silylated (V) carrier material (vide Table I)
D = Adsorption to non-silylated carrier material followed by 0.5% glutardialdehyde cross-linking* or 2.5% glutardialdehyde cross-linking**

| | A | B | C | D |
|---|---|---|---|---|
| Protein added per gram of carrier | 26 mg | 46 mg | 13mg | 20 mg |
| Activity added per gram of carrier | 610 U | 1070 U | 290 U b) | 760 U |
| Protein recovered in washings | 49% | 76% | 75% | 21% |
| Activity recovered in washings | 26% | 69% | 69% | 11% |
| Protein a) per gram of dry carrier | 14 mg | 11 mg | 3 mg | 16 mg |
| Immobilized activity per gram of dry carrier | 84 U | 45 U | ~0 | 133 U*<br>141 U** |
| Yield of immobilization based on non-recovered enzyme | 18% | 13% | ~0 | 20%*<br>21%** |

a) Calculated from non-recovered protein
b) By adjusting the pH of the enzyme solution to pH 4, 45% of the original enzyme amount was lost before the addition of silylated carrier material.

CLAIMS

1. An enzyme composition comprising an enzyme immobilized by attachment to a particulate inorganic carrier material substantially free from calcium carbonate, calcium oxide and acid soluble components, and comprising less than 1.2% by weight of trivalent metal ions, each particle of the inorganic carrier comprising at least 90% by weight of $SiO_2$ and

i) having a porosity of 25-50% by volume based on mercury porosimetry, corresponding to a void volume of about 0.15-0.45 $cm^3/g$ as determined by mercury porosimetry

ii) having an average pore radius of 0.02-0.1 µm

iii) having an abrasion resistance of at least 95%, preferably about 99%, as determined according to ASTM No. E 728-80.

2. An enzyme composition according to claim 1 wherein the carrier material has a surface area of at least 15 $m^2/g$, preferably at least 25 $m^2/g$, and more preferably at least 35 $m^2/g$, based on mercury porosimetry.

3. An enzyme composition according to claim 1 wherein the carrier material has a porosity of 30-45% by volume, preferably of 40-45% by volume, based on mercury porosimetry, corresponding to a void volume of about 0.18-0.36 $cm^3/g$, preferably 0.29-0.36 $cm^3/g$, as determined by mercury porosimetry.

4. An enzyme composition according to claim 3 wherein the carrier material has a porosity of about 42% by volume, based on mercury porosimetry, corresponding to about 0.32 $cm^3/g$ when measured by mercury porosimetry.

5. An enzyme composition according to claim 1 wherein the carrier material has an average pore radius of 0.03-0.05 µm, preferably about 0.05 µm.

6. An enzyme composition according to claim 1 wherein the carrier material is identical to a material prepared from a naturally occurring inorganic material comprising 20-50% of $CaCO_3$ and 80-50% of $SiO_2$, being partly crystalline and having an X-ray diffraction pattern with the following positions of the principal reflection peaks (in planar spacings, d(Å)): 4.328, 4.267, 4.092, 4.053, 3.860, 3.349, 3.040, 2.497, 2.283, 2.093, 1.912, 1.873, and furthermore

i) having a porosity of at least 10%, preferably at least 20%, by volume based on mercury porosimetry, corresponding to a void volume of about at least 0.05 $cm^3/g$, preferably at least 0.10 $cm^3/g$, as determined by mercury porosimetry

ii) having a broad pore radius distribution with pore radia from less than 0.0025 µm to about 5 µm, as determined by mercury porosimetry

iii) having a surface area of at least 10 $m^2/g$, preferably at least 16 $m^2/g$, based on mercury porosimetry,

by porosity-increasing treatment.

7. An enzyme composition according to claim 6 wherein the carrier material is identical to a material prepared from an inorganic material having the following X-ray diffraction pattern expressed by relative intensities as a function of the interplanar spacings ($I/I_{max}$, d(Å)): 23, 4.328; 25, 4.267; 35, 4.092; 36, 4.053; 15, 3.860; 4, 3.792; 54, 3.349; 100, 3.040; 4, 2.846; 1, 2.533; 23, 2.497; 5, 2.478; 5, 2.460; 23, 2.283; 2, 2.236; 4, 2.130; 20, 2.093; 2, 1.979; 7, 1.924; 20, 1.912; 22, 1.873; 7, 1.818, by porosity-increasing treatment.

8. An enzyme composition according to claim 1 wherein pores with radia of 0.005-1.0 µm in the carrier material account for at least 80%, preferably at least 90%, of the porosity.

9. An enzyme composition according to claim 1 wherein the carrier material has a particle size of at the most 6 mm.

10. An enzyme composition according to claim 1 wherein the carrier material has a hardness of about 7 according to the Mohs' scale.

11. An enzyme composition according to any of claims 1-10 wherein the enzyme is attached to the carrier by adsorption, optionally followed by intermolecular cross-linking, or by covalent bonding, optionally via a coupling agent.

12. An enzyme composition according to any of claims 1-11 wherein the enzyme is an oxidoreductase, a transferase, a hydrolase, a lyase, an isomerase or a ligase.

13. A method for immobilizing an enzyme comprising attaching the enzyme to a particulate inorganic carrier material free from calcium carbonate and calcium oxide and comprising less than 1.2% by weight of trivalent metal ions, each particle of the inorganic carrier comprising at least 90% by weight of $SiO_2$ and

i) having a porosity of 25-50%, preferably 30-45% by volume, more preferably 40-45% by volume, based on mercury porosimetry, corresponding to a void volume of about 0.15-0.45 $cm^3/g$, preferably about 0.18-0.36 $cm^3/g$, and more preferably 0.29-0.36 $cm^3/g$, as determined by mercury porosimetry

ii) having an average pore radius of 0.02-0.1 µm

iii) having an abrasion resistance of at least 95%, and more preferably about 99%, as determined according to ASTM No. E 728-80

14. A method according to claim 13 wherein the carrier material has a surface area of at least 15 $m^2/g$, preferably at least 25 $m^2/g$, and more preferably at least 35 $m^2/g$, based on mercury porosimetry.

15. A method according to claim 13 wherein the carrier material has a porosity of 30-45% by volume, preferably of 40-45% by volume, based

on mercury porosimetry, corresponding to a void volume of about 0.18-0.36 $cm^3/g$, preferably 0.29-0.36 $cm^3/g$, as determined by mercury porosimetry.

16. A method according to claim 15 wherein the carrier material has a porosity of about 42% by volume, based on mercury porosimetry, corresponding to about 0.32 $cm^3/g$ when measured by mercury porosimetry.

17. A method according to claim 13 wherein the carrier material has an average pore radius of 0.03-0.05 µm, preferably about 0.05 µm.

18. A method according to claim 13 wherein the attachment comprises an adsorption, preferably followed by cross-linking of the enzyme molecules.

19. A method according to claim 18 wherein the cross-linking is performed by means of glutardialdehyde.

20. A method according to claim 13 wherein the attachment is obtained via covalent bond(s) between the enzyme and the inorganic carrier.

21. A method according to claim 20 wherein the covalent bonding is performed via a coupling agent.

22. A method according to claim 13 wherein the attachment comprises silylating the carrier material and coupling the enzyme to the silylated material by means of a coupling agent.

23. A method according to claim 22 wherein the silylating agent is (3-aminopropyl)-triethoxysilane.

24. A method according to claim 23 wherein the amount of (3-aminopropyl)-triethoxysilane attached to the carrier is less than 100 mg per g of carrier material.

25. A method according to claim 22 wherein the coupling agent is selected from the group consisting of agents connecting an amino group on the silylated carrier material with an acid group from the enzyme or vice versa.

26. A method according to claim 25 wherein the coupling agent is a carbodiimide.

27. A method according to claim 22 wherein the coupling agent connects an amino group on the silylated carrier with an amino group on the enzyme or an acid group with an acid group.

28. A method according to claim 27 wherein the coupling agent is glutardialdehyde.

29. A method according to claim 13 wherein the attachment comprises coupling the enzyme to the carrier by means of cyanogenbromide.

30. A method according to any of claims 13-29 wherein the enzyme is an oxidoreductase, a transferase, a hydrolase, a lyase, an isomerase or a ligase.

31. A method for performing an enzymatic reaction comprising contacting an immobilized enzyme composition according to any of claims 1-12 with a substrate for the enzyme.

32. An inorganic carrier material substantially free from calcium carbonate, calcium oxide and acid soluble components, and comprising less than 1.2% by weight of trivalent metal ions, each particle of the inorganic carrier comprising at least 90% by weight of $SiO_2$ and

i) having a porosity of 25-50% by volume, based on mercury porosimetry, corresponding to a void volume of about 0.15-0.45 $cm^3/g$ as determined by mercury porosimetry

ii) having an average pore radius of 0.02-0.1 μm

iii) having an abrasion resistance of at least 95%, preferably about 99%, as determined according to ASTM No. E 728-80.

33. An inorganic carrier material according to claim 32 and having a surface area of at least 15 $m^2/g$, preferably at least 25 $m^2/g$, and more preferably at least 35 $m^2/g$, based on mercury porosimetry.

34. An inorganic carrier material according to claim 32 and having a porosity of 30-45% by volume, preferably of 40-45% by volume, based on mercury porosimetry, corresponding to a void volume of about 0.18-0.36 $cm^3/g$, preferably 0.29-0.36 $cm^3/g$, as determined by mercury porosimetry.

35. An inorganic carrier material according to claim 32 and having a porosity of about 42% by volume, based on mercury porosimetry, corresponding to about 0.32 $cm^3/g$ when measured by mercury porosimetry.

36. An inorganic carrier material according to any of claims 32-35 and having a mean pore radius of 0.03-0.05 µm, preferably about 0.05 µm.

37. An inorganic carrier material according to any of claims 32-36 which is identical to a material prepared from an inorganic material comprising 20-50% of $CaCO_3$ and 80-50% of $SiO_2$, being partly crystalline and having an X-ray diffraction pattern with the following positions of the principal reflection peaks (in planar spacings, d(Å)): 4.328, 4.267, 4.092, 4.053, 3.860, 3.349, 3.040, 2.497, 2.283, 2.093, 1.912, 1.873, and furthermore

i) having a porosity of at least 10%, preferably at least 20%, by volume based on mercury porosimetry, corresponding to a void volume of about at least 0.05 $cm^3/g$, preferably at least 0.10 $cm^3/g$, as determined by mercury porosimetry

ii) having a broad pore radius distribution with pore radia from <0.0025 µm to about 5 µm, as determined by mercury porosimetry

iii) having a surface area of at least 10 $m^2/g$, preferably at least 16 $m^2/g$, based on mercury porosimetry,

by porosity-increasing treatment.

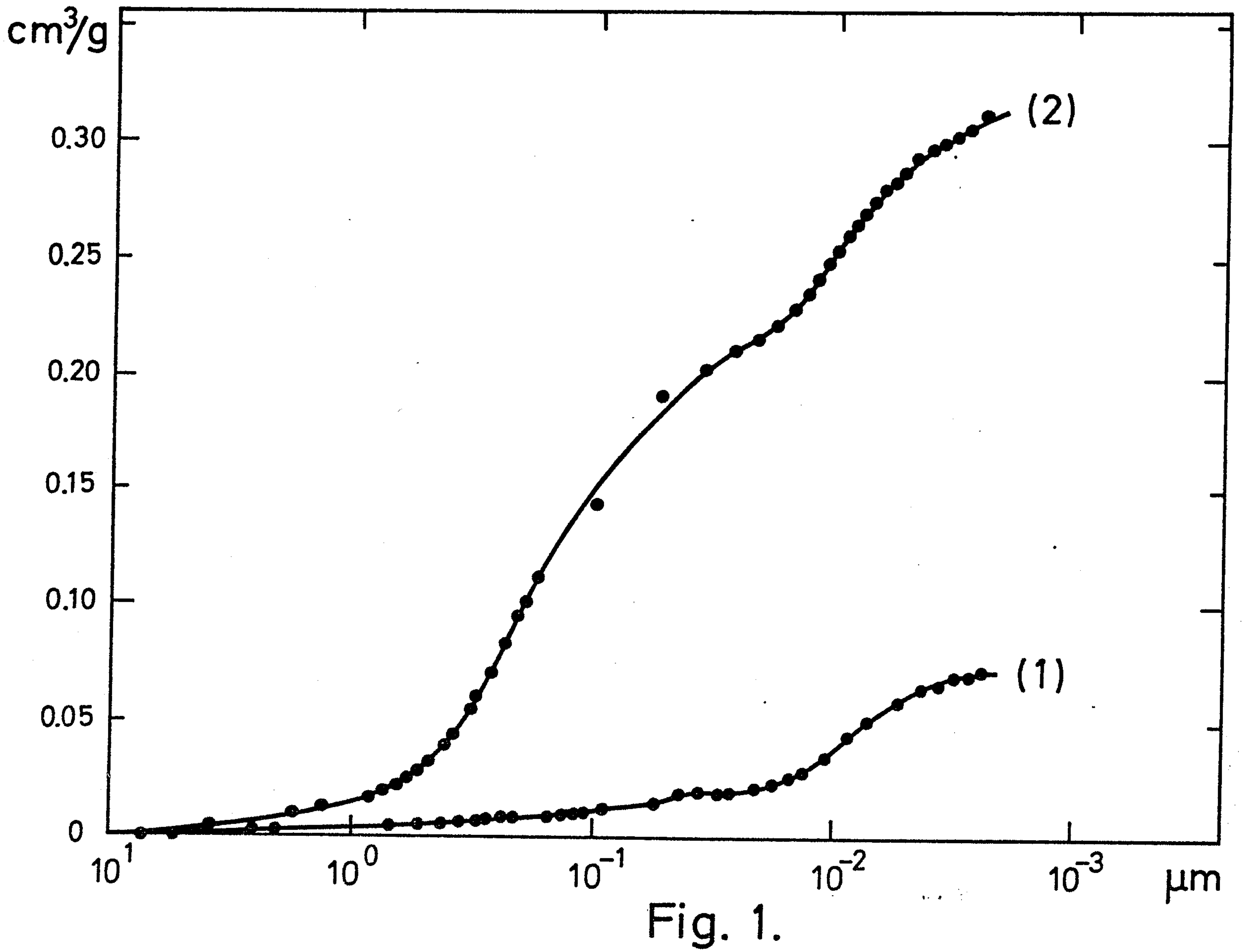


Fig. 1.

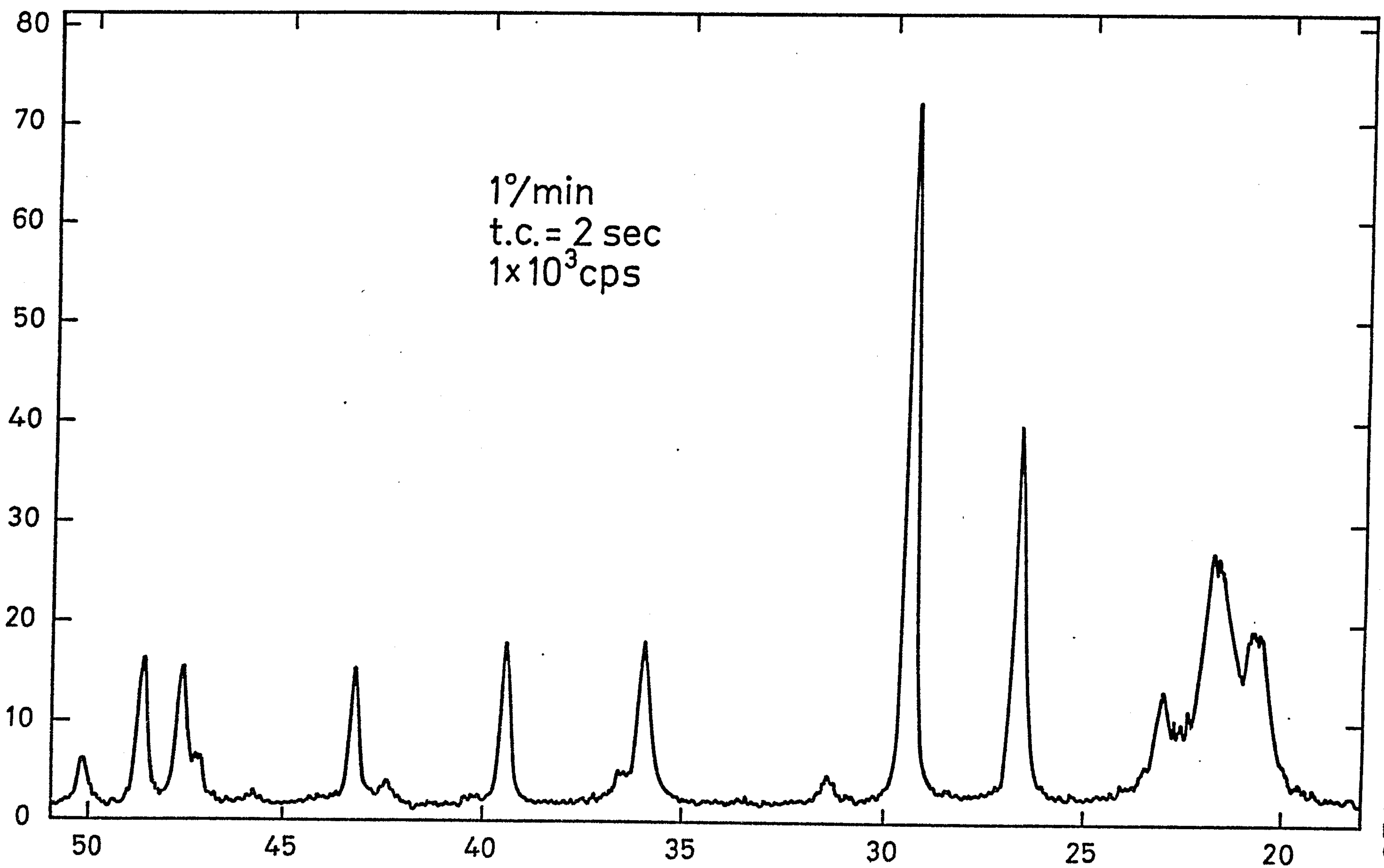

1°/min
t.c. = 2 sec
$1 \times 10^3$ cps
80
70
60
50
40
30
20
10
0
50
45
40
35
30
25
20


Fig. 2.

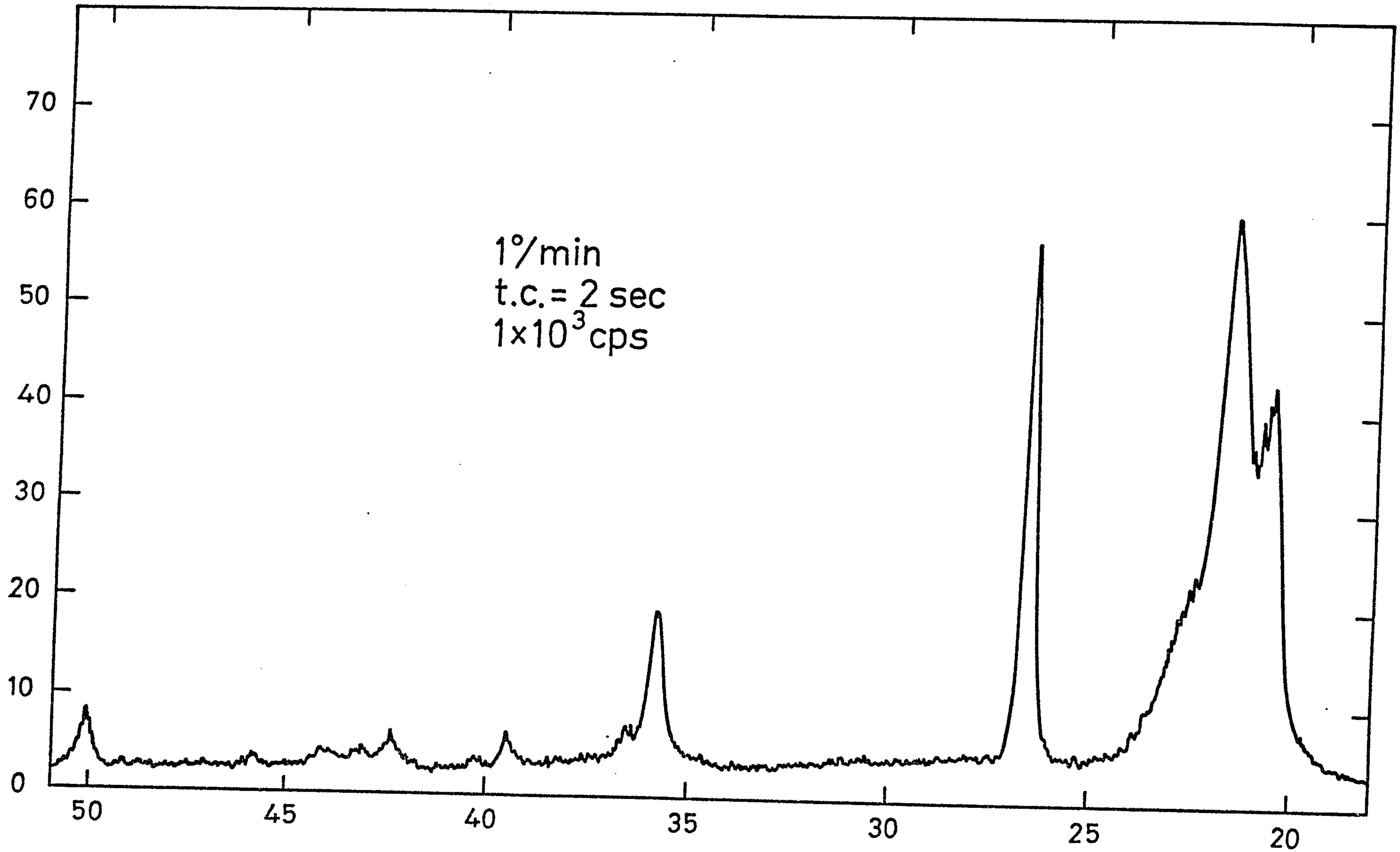


Fig. 3.

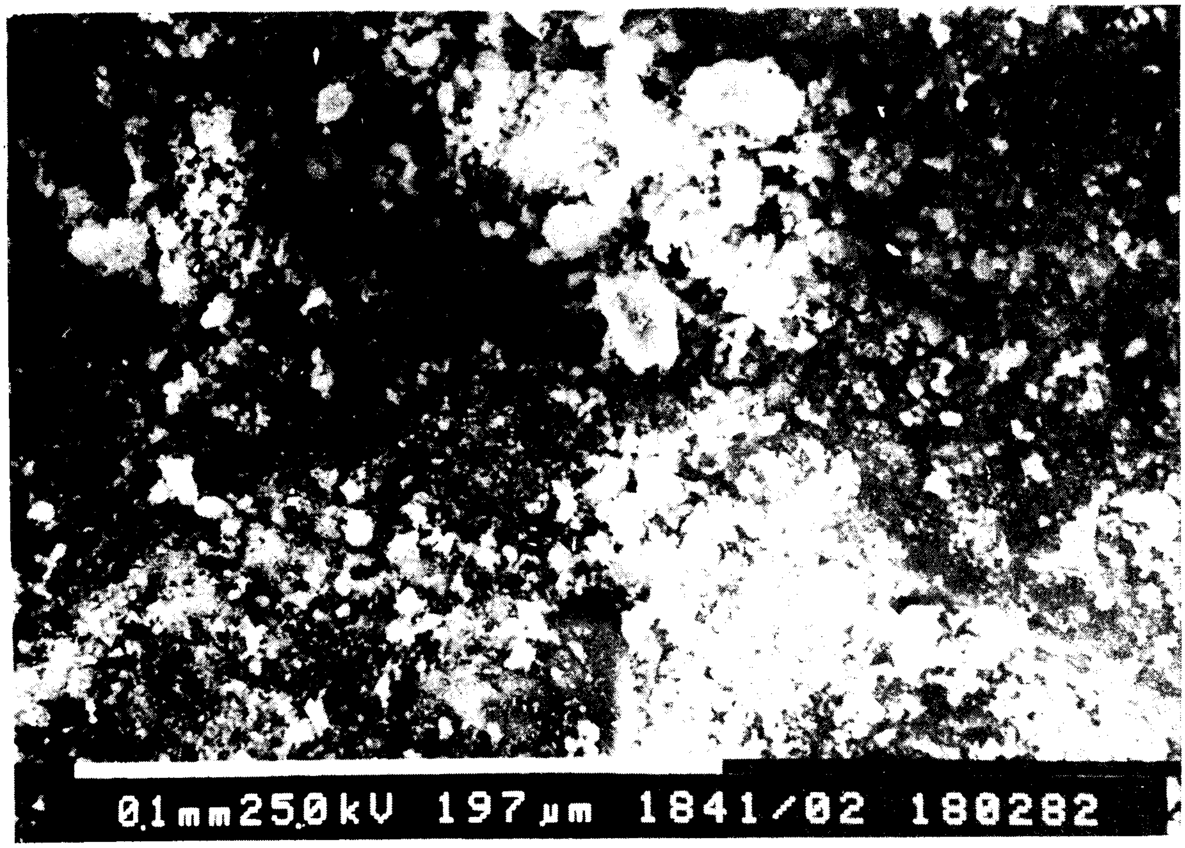
0,1mm25,0kV 197µm 1841/02 180282

Fig. 4

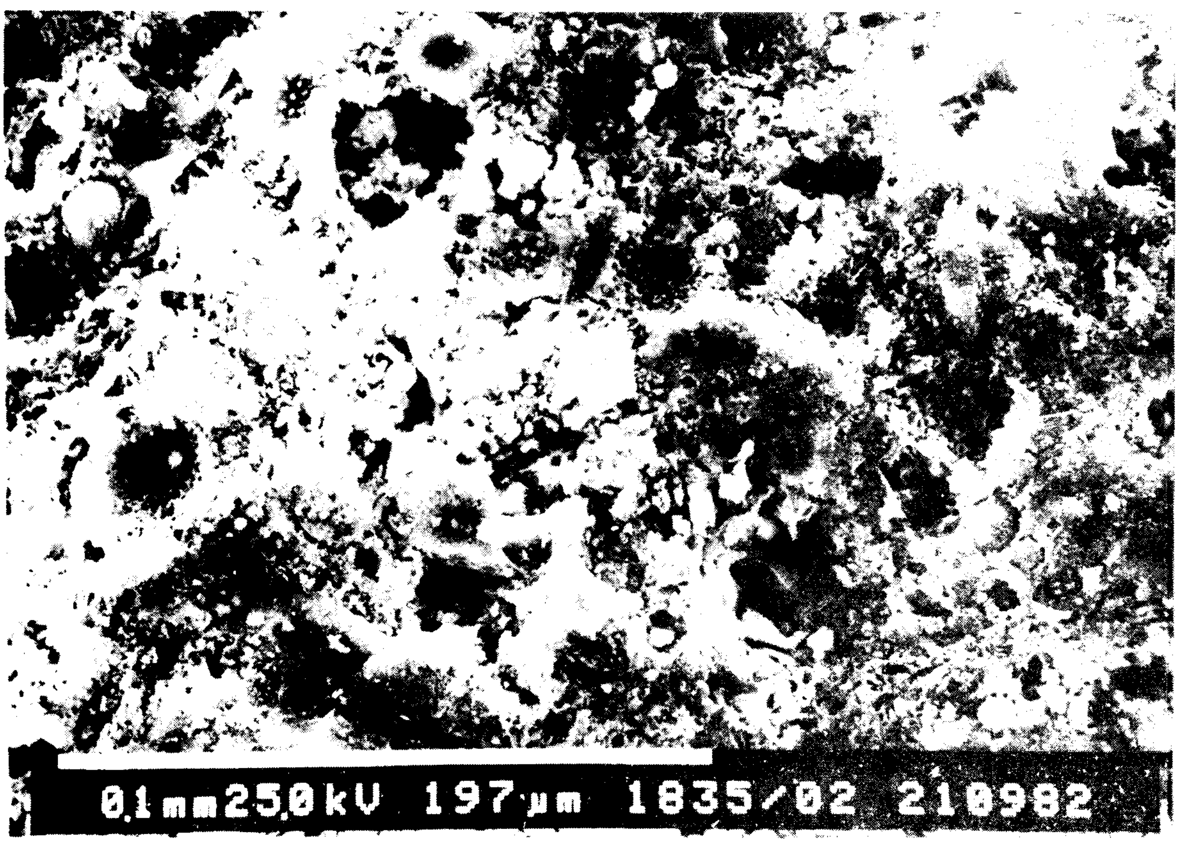
0,1mm25,0kV 197µm 1835/02 210982

Fig. 5

European Patent Office

# EUROPEAN SEARCH REPORT

0131251

Application number

EP 84107778.7

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| D,A | US - A - 4 384 045 (HO et al.)<br>* Column 3, lines 13-40; column 4, lines 1-6; column 5, line 3 - column 6, line 2; example 1; table 1 *<br>-- | 1,9,11, 13,18, 19,20, 22,23, 25,26, 28,32 | C 12 N 11/14<br>C 01 B 33/12 |
| A | GB - A - 1 550 128 (AMERACE)<br>* Page 2, lines 12-24; page 5, lines 4-35; claims *<br>-- | 1,11,12, 13,19, 20,21, 22,23, 25,28, 32 | |
| D,A | US - A - 3 669 841 (MILLER)<br>* Column 1, lines 57-63; column 2, lines 28-45; column 3, lines 38-54; example 1 *<br>-- | 1,12,13, 19,20, 21,22, 23,24, 28,32 | TECHNICAL FIELDS SEARCHED (Int. Cl.4) |
| A | DE - A - 2 106 214 (MONSANTO)<br>* Page 10, line 17 - page 11, line 22; claims *<br>-- | 1,13,20, 21,22, 23,26, 32 | C 12 N<br>C 01 B |
| D,A | US - A - 3 519 538 (MESSING)<br>* Column 2, line 31 - column 3, line 11; column 4, lines 34-47 *<br>-- | 1,12,13, 20,21, 22,23, 32 | |
| A | GB - A - 1 530 369 (RHONE-POULENC)<br>* Page 1, lines 15-22 *<br>---- | 1,13,26, 28,32 | |

The present search report has been drawn up for all claims

| Place of search | Date of completion of the search | Examiner |
|---|---|---|
| VIENNA | 27-09-1984 | BECKER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document